# EUROPÄISCHE PATENTSCHRIFT

(11) **EP 0 543 122 B1**
(45) Veröffentlichungstag und Bekanntmachung des Hinweises auf die Patenterteilung: **26.06.1996**
(21) Anmeldenummer: 92116830.8
(22) Anmeldetag: 01.10.1992
(51) Int. Cl.: A61B 17/39

(54) **Medizinisches Hochfrequenz-Koagulations-Schneidinstrument**
H.F. electrosurgical cutting and coagulating instrument
Appareil chirurgical pour la coagulation et la coupe à haute fréquence

(30) Priorität: 19.11.1991 DE 4138116
(43) Veröffentlichungstag der Anmeldung: 26.05.1993
(73) Patentinhaber: DELMA ELEKTRO-UND MEDIZINISCHE APPARATEBAU GESELLSCHAFT mbH, D-78532 Tuttlingen (DE)
(72) Erfinder: Hagen, Alfred, W-7200 Tuttlingen/Nendingen (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- EP-A- 0 210 125
- EP-A- 0 280 972
- CH-A- 547 103
- DE-A- 2 415 263
- US-A- 4 011 872
- US-A- 4 834 095

## Beschreibung

Die Erfindung betrifft ein medizinisches Hochfrecuenz-Koagulations-Schneidinstrument nach dem Oberbegriff des Patentanspruchs 1.

Ein derartiges Instrument ist aus der CH-A-547103 bekannt. Bei einer Ausführungsform dieses bekannten Instrumentes ist der Leitungsstab für die Schneidelektrode konzentrisch innerhalb eines Leitungsrohres angeordnet, welches eine Koagulationszange trägt. Das Koagulieren und elektrische Schneiden wird mit einer Neutral-Elektrodenplatte durchgeführt, die an die Haut des Patienten angeschlossen ist. Bei einer weiteren Ausführungsform können anstelle von zwei Koagulationszangen mit Neutralelektrode zwei voneinander isolierte Koagulationszangen für die bipolare Koagulation eingesetzt werden, welche über den zentralen Leitungsstab und das eine Leitungsrohr mit Hochfrequenzenergie gespeist werden. Bei dieser Ausführungsform ist die Anordnung einer Schneidelektrode nicht möglich.

Weiter ist bereits eine Koagulationszange bekannt (DE-PS 27 34 847), bei der die beiden Koagulationselektroden durch Vorschieben eines Rotors mittels des Betätigungskopfes zangenförmig aufeinander zu bewegt werden können. Eine Schneidelektrode ist bei diesem Instrument nicht vorgesehen.

Schließlich ist es aus der nicht-vorveröffentlichten Offenlegungsschrift 41 22 219 bekannt, zwei Koagulationselektroden und deren Zuleitung als koaxial zueinander angeordnete Rohre auszubilden und dazwischen eine axial verstellbare Schneidelektrode anzuordnen, die über einen innerhalb der Rohre isoliert angeordneten Leitungsstab mit Hochfrequenzspannung versorgt wird.

Die Aufgabe der Erfindung besteht darin, ein weiteres medizinisches Koagulations-Schneidinstrument zur Verfügung zu stellen. Insbesondere soll das erfindungsgemäße Koagulations-Schneidinstrument eine Fülle unterschiedlicher Koagulations- und Schneidaufgaben lösen können. Es soll zwecks Reinigung auch leicht zerlegbar sein.

Zur Lösung dieser Aufgabe sind die Merkmale des kennzeichnenden Teils des Patentanspruchs 1 vorgesehen.

Aufgrund dieser Ausbildung wird zum einen eine sehr stabile Ausbildung des Instrumentenschafts gewährleistet, während zum anderen die für die Beaufschlagung der Koagulationselektroden und der Schneidelektrode erforderlichen Hochfrequenzspannungen und -ströme mittels einer sehr kompakten und stabilen Zuleitungsanordnung zugeführt werden können. Die Schneidelektrode wird dabei mit einer zum Schneiden geeignete Hochfrequenzspannung versorgt, wobei die beiden Koagulationselektroden zu einer Neutralelektrode zusammengeschaltet sind. Im anderen Schaltzustand ist die Schneidelektrode in den Instrumentenschaft zurückgezogen oder abmontiert, wobei dann die beiden Koagulationselektroden mit einem Hochfrequenz-Koagulationsstrom gespeist werden, der durch Anlegen der beiden stationären Koagulationselektroden an Gewebe die gewünschte Erhitzung bewirkt.

Der axial bewegliche Leitungsstab kann massiv oder aber auch rohrförmig hohl oder als zusammengesetzter Körper ausgebildet sein. Er ist vorzugsweise mit einer Isolierhülle überzogen und von einem schmalen Spalt umgeben, damit die relative Axialverschieblichkeit gewährleistet ist.

Für das Erfassen von zu koagulierendem oder zu schneidendem Gewebe ist die Ausführungsform nach Anspruch 2 besonders zweckmäßig.

Bei der Ausführungsform nach Anspruch 3 kann die Schneidelektrode vorzugsweise zwischen den Koagulationselektroden axial bewegt werden.

Für den Schneidvorgang in diesem Fall ist besonders zweckmäßig die Ausführungsform nach Anspruch 4.

Es ist jedoch auch möglich, die Schneidelektrode gemäß Anspruch 5 verschwenkbar anzuordnen. Vorteilhafte Weiterbildungen dieser Ausführungsform sind den Ansprüchen 6 bis 9 zu entnehmen.

Bevorzugte Ausbildungen des Betätigungskopfes sind durch die Ansprüche 10 bis 16 gekennzeichnet.

Besonders vorteilhaft ist es, wenn das Instrumentenschaft vom vorderen Ende des Betätigungskopfes abziehbar und außerdem der feststehende und der bewegliche Teil des Betätigungskopfes voneinander trennbar sind. Dann können die einzelnen Teile bequem jedes für sich gesäubert und auch gegebenenfalls sterilisiert werden.

Die Erfindung wird im folgenden beispielsweise anhand der Zeichnung beschrieben; in dieser zeigt:
- Fig. 1: eine teilweise geschnittene Seitenansicht des Betätigungskopfes des erfindungsgemäßen Koagulations-Schneidinstrumentes,
- Fig. 2: einen Schnitt nach Linie II-II in Fig. 1,
- Fig. 3: einen zweifach aufgebrochenen Axialschnitt durch den Instrumentenschaft des erfindungsgemäßen Koagulations-Schneidinstrumentes,
- Fig. 4: eine gegenüber Fig. 3 um 90° um die Achse des Instrumentenschaftes gedrehte Schnittansicht des vorderen Teils des Instrumentenschafts nach Fig. 3,
- Fig. 5: eine zu Fig. 3 analoge Schnittansicht des vorderen Teils des Instrumentenschafts mit einer anderen Ausführungsform des Schneidinstrumentes, und
- Fig. 6: eine um 90° um die Achse des Instrumentenschaftes gedrehte Ansicht des Gegenstandes der Fig. 5.

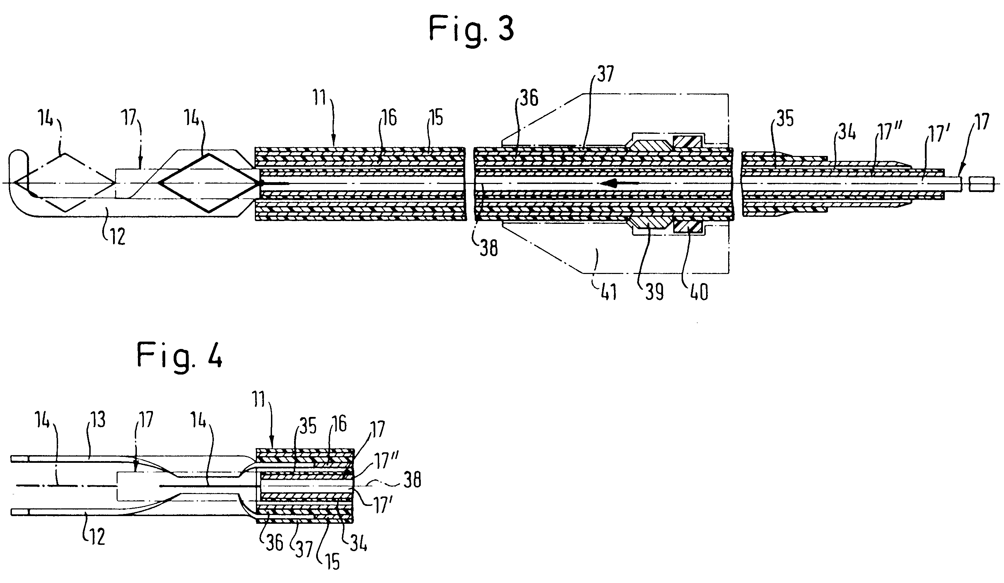

Nach den Fig. 3 und 4 besteht ein einen über den größten Teil seiner Länge konstanten kreisrunden Querschnitt aufweisender Instrumentenschaft 11 radial von innen nach außen gehend aus folgenden zur Mittelachse 38 konzentrischen Bestandteilen:
- einem mit einer Isolierhülle 34 überzogenen Leitungsstab 17, der aus einer zentralen Stange 17' und einem auf diese fest aufgesetzten Rohr 17'' besteht und aus der in Fig.3 gezeigten Ruhestellung in Pfeilrichtung in eine strichpunktiert angedeutete vordere Endstellung ausfahrbar ist;
- einem die axiale Verschiebung des Leitungsstabes 17 gewährleistender Spalt 35;
- einem Leitungsrohr 16;
- einer Isolierschicht 36,
- einem weiteren Leitungsrohr 15;und
- einer Außenisolierschicht 37.

Am proximalen, d.h. dem Patienten zugewandten Ende des Leitungsstabes 17, das in der Ruhestellung im wesentlichen mit dem vorderen Ende des Instrumentenschafts 11 zusammenfällt, ist eine rhombusförmige Drahtbügel-Elektrode angeordnet, die mit einem zur Durchführung eines Schneidvorganges geeigneten Hochfrequenzstrom gespeist werden kann. Die vorderen Enden der Leitungsrohre 15, 16 sind gemäß Fig. 4 mit einer ersten feststehenden Koagulationselektrode 12 bzw. einer zweiten feststehenden Koagulationselektrode 13 verbunden. Gemäß den Fig. 3 und 4 erstrecken sich die beiden länglichen und flachen Koagulationselektroden 12, 13 im wesentlichen parallel zur Mittelachse 38 und bilden an ihrem proximalen Ende einen z.B. rechtwinklig abgewinkelten Haken. Im rückwärtigen Bereich sind die Koagulationselektroden 12, 13 teilzylinderartig ausgebildet, um einen stetigen Übergang zu den Leitungsrohren 15, 16 herzustellen, an denen sie befestigt und mit denen sie elektrisch verbunden sind. Die Schneidelektrode 14 erstreckt sich symmetrisch zwischen die beiden Koagulationselektroden 12, 13 und ist durch Nach-Vorn-Verschieben des Leitungsstabes 17 aus der in den Fig. 3 und 4 in ausgezogenen Linien dargestellten Position in die in strichpunktierten Linien wiedergegebene Stellung verschiebbar.

Im distalen Endbereich des Instrumentenschaftes 11 ist in der Außenisolierschicht 37 ein Befestigungsflansch 39 und dahinter eine Ringdichtung 40 vorgesehen. In diesem Bereich befindet sich auf dem Instrumentenschaft 11 eine Überwurfmutter 41, die in Fig. 3 nur schematisch strichpunktiert angedeutet ist.

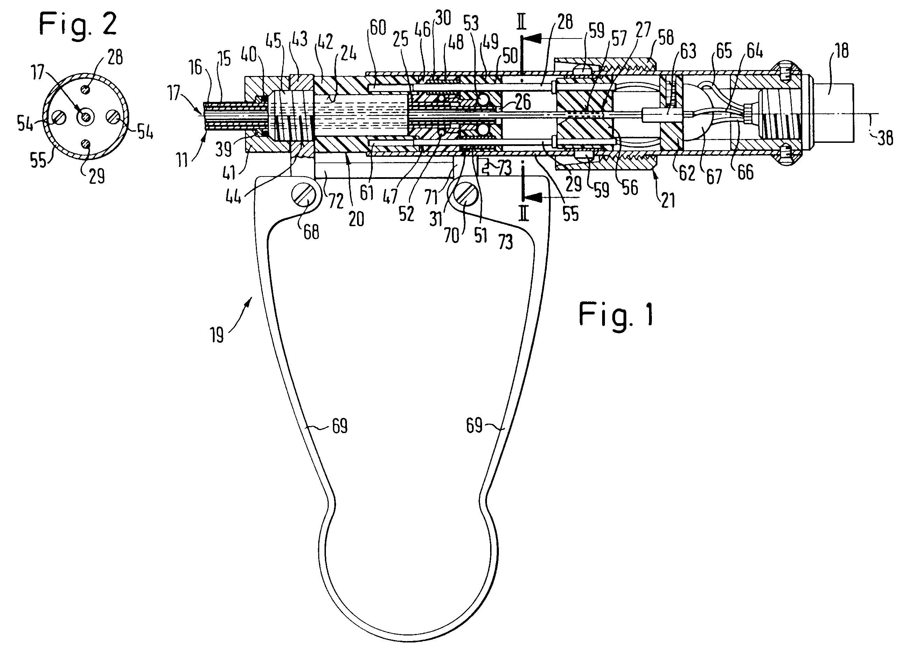

Hinter der Überwurfmutter 41 enden die verschiedenen koaxialen Rohre abgestuft von außen nach innen bei immer größeren Längen, so daß die Leitungsrohre 15, 16 und auch der zentrale Leitungsstab 17 mit größeren Teilen ihrer Umfänge freiliegen und in der im folgenden anhand der Fig. 1 und 2 zu beschreibenden Weise mit dem Betätigungskopf 19 in eine feste mechanische und elektrische Verbindung gebracht werden zu können.

Es ist darauf hinzuweisen, daß der Grad des Überstehens der einzelnen koaxialen Elemente in Fig. 3 in einem anderen Maßstab als in Fig. 1 dargestellt ist.

Nach Fig. 1 ist das distale Ende des Instrumentenschafts 11 in eine zentrale Aufnahmebohrung 24 des feststehenden Teils 20 des Betätigungskopfes 19 eingeführt. Dessen feststehendes Teil 20 besteht im wesentlichen aus einem Kunststoffrohr 42, an dessen Frontseite ein vorzugsweise metallisches Endstück 43 angebracht ist, welches eine zentrale Bohrung 44 aufweist, in der eine vorn vorstehende Gewindehülse 45 befestigt ist. Die Überwurfmutter 41 ist auf das Außengewinde dieser Hülse 45 axial aufgeschraubt und drückt dadurch den Flansch 39 über die Dichtung 40 gegen die Frontseite der Gewindehülse 45. Auf diese Weise ist das Instrumentenschaft 11 lösbar am Betätigungskopf 19 befestigt.

Hinter dem Isolierrohr 42 ist in axialer Ausrichtung mit ihm eine konzentrische Kontakthülse 46 aus Isoliermaterial angeordnet, die exzentrisch an diametral gegenüberliegenden Stellen parallel zur Achse 38 verlaufende Bohrungen 30, 47 aufweist, von denen die obere in Fig. 1 mit einer Metallhülse 48 ausgekleidet ist, so daß hier eine Kontaktbohrung 30 vorliegt.

Hinter der isolierenden Kontakthülse 46 ist in axialer Ausrichtung mit dieser eine weitere isolierende Kontakthülse 49 angeordnet und an ihr befestigt, die mit den Bohrungen 30, 47 axial ausgerichtete Bohrungen 31, 50 aufweist. Nur die in der Fig. 1 oberen Bohrung 30 diametral gegenüberliegende Bohrung 31 ist wieder mit einer Metallhülse 51 ausgekleidet.

Während die Kontakthülse 46 einen zentralen Kontaktkanal 25 zur Aufnahme des Leitungsrohres 15 aufweist, ist die Kontakthülse 49 mit einem zentralen Kontaktkanal 26 kleineren Durchmessers zur Aufnahme des Leitungsrohres 16 versehen.

In der Wand der Kontakthülsen 46, 49 angeordnete Federringe 52, 53 gewährleisten eine leitende Verbindung zwischen den Leitungsrohren 15, 16 einerseits und den Metallhülsen 48, 51 andererseits.

Das Isolierrohr 42 und die damit ausgerichteten Kontakthülsen 46, 48, die durch aus Fig. 2 ersichtliche Schrauben 54 axial miteinander verbunden sind, haben eine gemeinsame zylindrische Außenkontur, so daß darauf eine metallische Führungshülse 55 axial geführt verschiebbar angebracht werden kann.

Die Führungshülse 55 trägt in ihrem Mittelbereich einen aus Isoliermaterial bestehenden Klemmblock 56 mit einer geschlitzten Zentralbohrung 57, durch die hindurch sich das hintere Ende des Leitungsstabes 17 bzw. der leitenden Stange 17' erstreckt. Durch eine konische Überwurfmutter 58 und relativ zur Führungshülse 55 axial fest und radial verschiebliche Druckstifte 59, die mit dem Klemmblock 56 verbunden sind, kann der Leitungsstab 17 lösbar im Klemmblock 56 befestigt werden, so daß insgesamt eine lösbare Befestigungsvorrichtung 27 für den Leitungsstab 17 vorliegt.

In dem Klemmblock 56 sind exzentrisch und in Ausrichtung mit den Bohrungen 30, 50 bzw. 31, 47 elektrisch leitende Kontaktstifte 28, 29 fest angeordnet, die im Gleitsitz in die Bohrungen 30, 50 bzw. 31, 47 eingreifen.

Um einen ausreichenden relativen Verschiebungsbereich der Kopfteile 20, 21 zu gewährleisten, erstrecken sich die Bohrungen vorzugsweise auch noch in der dargestellten Weise in Bereiche 60, 61 des Isolierrohres 42 hinein.

Das hintere Ende des Leitungsstabes 17 berührt einen in einer isolierenden Zwischenwand 62 angeordneten Kontaktstift 63, welcher über eine Leitung 64 mit einer am Ende der Führungshülse 55 vorgesehenen Anschlußkupplung 18 verbunden ist.

Zwei weitere Kabel 65, 66 führen ggf. über einen Kondensator 67 zu den beiden Kontaktstiften 28, 29.

Die vordere Endplatte 43 ist über eine Schraube 68 um eine Querachse schwenkbar mit einem federnden Betätigungsgriff 69 verbunden, dessen anderer Schenkel über eine Schraube 70 mit einem an der Führungshülse 55 befestigten Führungsblock 71 verbunden ist, der auf einer sich von der Endplatte 43 parallel zur Achse 38 erstreckenden Führungsstange 72 axial verschiebbar gelagert ist.

Die Führungsstange 72 ist in eine entsprechende Gewindebohrung der Endplatte 43 eingeschraubt, und ein Schraubenkopf 73 begrenzt die durch die Federkraft des Griffes 69 herbeigeführte axiale Auseinanderbewegung der Führungshülse 55 sowie des Isolierrohrs 42.

Das Instrument läßt sich dadurch zerlegen, daß nach Abschrauben der Überwurfmutter 41 und Lösen der Überwurfmutter 58 zunächst der Instrumentenschaft 11 aus dem Betätigungskopf 19 herausgezogen wird. Dann wird der Betätigungsgriff 69 durch Lösen der Schrauben 68, 70 abgeschraubt und die Führungsstange 72 durch Herausdrehen mittels des Schraubenkopfes 73 entfernt. Nunmehr kann das bewegliche Kopfteil 21 von dem festen Kopfteil 20 axial abgezogen werden, wobei die Kontaktstifte 28, 29 aus den zugeordneten Bohrungen 30, 50, 60 bzw. 31, 47, 61 axial herausgezogen werden. Es können jetzt alle notwendigen Reinigungs- und Reparaturarbeiten an dem Betätigungskopf 19 durchgeführt werden.

Zum erneuten Zusammensetzen des Instrumentes werden zunächst die beiden Kopfteile 20, 21 mit dem Betätigungsgriff 69 zum Betätigungskopf 19 zusammengesetzt. Nun wird der in den Fig. 3 und 4 im einzelnen gezeigte Instrumentenschaft 11 in der in Fig.1 schematisch gezeigten Weise mit seinem distalen Ende in die Aufnahmebohrung 24 eingesteckt, bis der Leitungsstab 17 die Bohrung 57 durchgreift und am Kontaktstift 63 anliegt sowie die Leitungsrohre 15, 16 mit den leitenden Federringen 52, 53 in elektrischen Kontakt kommen. Dann wird das Instrumentenschaft 11 durch Aufschrauben der Überwurfmutter 41 auf die Gewindehülse 45 endgültig am Betätigungskopf 19 befestigt.

Durch Zusammendrücken des federnden Handgriffes 69 wird der bewegliche Kopfteil 21 in Richtung des feststehenden Kopfteils 20 verschoben, wobei der Leitungsstab 17 relativ zu den Leitungsrohren 15, 16 nach vorne bewegt wird, so daß die rhombusförmige Schneidelektrode 14 nach den Fig.3 und 4 aus der in ausgezeichneten Linien dargestellten Position in die vordere, in strichpunktierten Linien angedeutete Stellung verschoben wird. Die Kontaktstifte 28, 29 verschieben sich dabei unter Aufrechterhaltung des elektrischen Kontaktes in den Kontaktbohrungen 30, 31.

Durch Entspannen des federnden Handgriffs 69 bewegen sich die beiden Kopfteile 20, 21 wieder auseinander in die aus den Fig. 1, 3 und 4 ersichtliche Ausgangsposition.

Im zurückgezogenen Zustand liegt die Schneidelektrode 14 nach den Fig. 3 und 4 zwischen den am Ende teilzylindrisch gebogenen Koagulationselektroden 12, 13. In diesem Zustand können mit dem Instrument durch Anlegen eines geeigneten Hochfrequenzstroms an die Koagulationselektroden 12, 13 Koagulationen durchgeführt werden.

Soll ein Schneidvorgang mit der Schneidelektrode 14 ausgeführt werden, so betätigt der Chirurg einen nicht gezeigten Umschalter, wodurch ein nicht gezeigtes Steuergerät die beiden Koagulationselektroden 12, 13 zu einer einzigen Neutralelektrode zusammenschaltet, während die Schneidelektrode 14 bei Betätigung eines Schalters an einen einen zum Schneiden geeigneten Hochfrequenzstrom angelegt wird. Mittels der hakenförmigen Koagulationselektroden 12, 13 ergriffene Gefäße oder Gewebeteile können so mit der Schneidelektrode 14 zerschnitten werden.

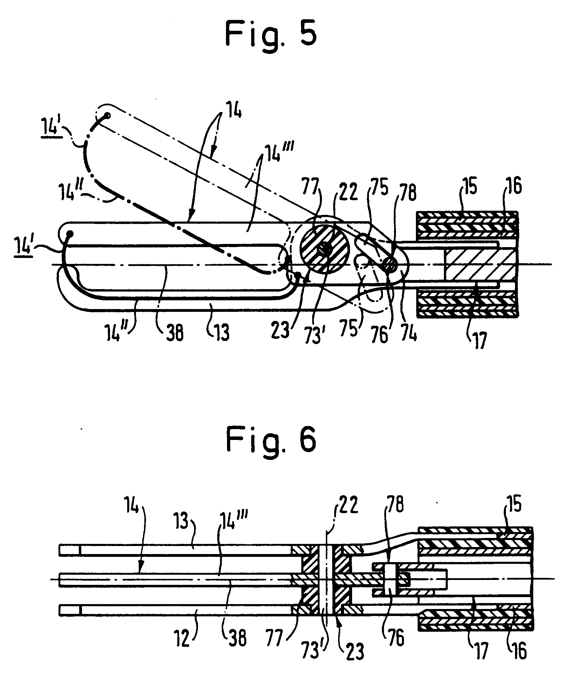

Nach den Fig. 5 und 6, in denen gleiche Bezugszahlen entsprechende Bauteile wie in den vorangehenden Figuren bezeichnen, weist die Schneidelektrode 14 einen um eine Querachse 22 verschwenkbaren Tragarm 14''' auf, an dem ein Drahtbügel 14' angeordnet ist, der sich im heruntergeklappten Zustand mit einem geradlinigen Teil 14'' zwischen die beiden Koagulationselektroden 12, 13 erstreckt. Der Teil 14'' kann auch wellenförmig bzw. zick-zack-förmig ausgebildet sein. Auch eine gebogene Form ist möglich.

Das Lager 23 für die Querachse 22 besteht aus einem einen Metallstift 73' enthaltenden Isolationsblock 77, der im hinteren Bereich der Koagulationselektroden 12, 13 angeordnet ist und sich zwischen ihnen erstreckt.

Ein sich hinter die Querachse 22 erstreckender Betätigungsarm 74 der Schneidelektrode 14 ist mit einem schräg zur Achse 38 verlaufenden Langloch 75 versehen, in welches ein Querstift 76 im Kulisseneingriff erstreckt, der am vorderen Ende des Leitungsstabes 17 befestigt ist. Das Langloch 75 und der Querstift 76 bilden ein Getriebe 78, das eine Linear- in eine Schwenkbewegung umsetzt. Durch Vorschieben des Leitungsstabes 17 aus der in Fig. 5 in ausgezogenen Linien dargestellten Position wird somit auf die Schneidelektrode 14 eine Schwenkbewegung aus der in Fig.5 in ausgezogenen Linien dargestellten Position in die in strichpunktierten Linien wiedergegebene Lage hervorgerufen. Für die Erzeugung einer Schwenkbewegung aus einer Linearbewegung des Leitungsstabes 17 können auch andere geeignete Getriebe verwendet werden.

Das Ausführungsbeispiel nach den Fig. 5 und 6 arbeitet beim Koagulieren wie die Ausführungsform nach Fig. 3,4, beim Schneiden dagegen in folgender Weise:

Zunächst wird bei gedrücktem Betätigungsgriff 69 (Fig.1), d.h. im offenen Zustand der Schneidelektrode 14 (strichpunktierte Darstellung in Fig. 5) ein Gefäß oder Gewebeteil mit den hakenförmigen Koagulationselektroden 12, 13 ergriffen, die in diesem Fall als Neutralelektrode geschaltet sind. Sobald dies geschehen ist, wird die Schneid-Hochfrequenzspannung an die Schneidelektrode 14 angelegt, d.h. ein Lichtbogen zwischen dem Drahtbügel 14' und den Koagulationselektroden 12, 13 erzeugt, und der Leitungsstab 17 wird beispielsweise durch Entspannung des Handgriffs 69 nach Fig. 1 zurückgezogen, wobei der Drahtbügel 14' zwischen die Koagulationselektroden 12, 13 schwenkt und dabei das betreffende Gewebeteil sauber durchschneidet.

## Patentansprüche

1. Medizinisches Hochfrequenz-Koagulations-Schneidinstrument mit einem Instrumentenschaft (11), an dessen proximalem Ende zwei feststehende Koagulationselektroden (12, 13) und eine bewegliche Schneidelektrode (14) vorgesehen sind, sowie mit einem am distalen Ende des Instrumentenschafts (11) angeordneten Betätigungskopf (19), welcher aus einem am Instrumentenschaft (11) befestigten festen Kopfteil (20) und einem relativ dazu beweglichen Kopfteil (21) besteht, wobei eine Relativbewegung der beiden Kopfteile (20, 21) auf die Schneidelektrode (14) übertragen wird, die Koagulationselektroden (12, 13) sowie die Schneidelektrode (14) zwecks Verbindung mit einem Hochfrequenz-Versorgungsgerät über im Instrumentenschaft (11) isoliert zueinander verlegte Hochfrequenz-Zuführleitungen (15, 16, 17) zu einem vorzugsweise am distalen Ende vorgesehenen Kabelanschluß (18) geführt sind und das Hochfrequenz-Versorgungsgerät zumindest zwei Schaltstellungen aufweist, in einer von denen die Schneidelektrode (14) abgeschaltet ist und die Koagulationselektroden (12, 13) mit einem Koagulations-Hochfrequenzstrom beaufschlagt sind und in der anderen von denen eine oder beide Koagulationselektroden (12, 13) als Neutralelektrode geschaltet sind und an die Schneidelektrode (14) und die Neutralelektrode eine einen Schneidlichtbogen ermöglichende Hochfrequenzspannung angelegt ist, wobei die Hochfrequenz-Zuführleitung für die eine Koagulationselektrode ein Leitungsrohr ist, innerhalb dessen die Hochfrequenz-Zuführleitung für die Schneidelektrode (14) isoliert als axialer und durch den Betätigungskopf (19) axial beweglicher Leitungsstab (17) angeordnet ist,
dadurch **gekennzeichnet,**
daß die Hochfrequenz-Zuführleitung für die zweite Koagulationselektrode ebenfalls als ein den Leitungsstab (17) umgebendes zweites Leitungsrohr ausgebildet ist und beide Leitungsrohre (15, 16) ineinander und vorzugsweise konzentrisch zueinander und zum Instrumentenschaft (11) angeordnet sind.

2. Instrument nach Anspruch 1, dadurch gekennzeichnet, daß die Koagulationselektroden als zwei zumindest im wesentlichen parallel zueinander verlaufende Hakenelektroden (12, 13) ausgebildet sind, zwischen denen die Schneidelektrode (14) beweglich angeordnet ist.

3. Instrument nach Anspruch 1 oder 2, dadurch gekennzeichnet, daß die Schneidelektrode (14) am proximalen Ende des Leitungsstabes (17) befestigt und durch den mittels des Betätigungskopfes (19) verschiebbaren Leitungsstab (17) relativ zu den Koagulationselektroden (12, 13) axial verschiebbar ist.

4. Instrument nach einem der vorhergehenden Ansprüche, dadurch gekennzeichnet, daß die Schneidelektrode (14) als Drahtbügel, insbesondere rhombischer Draht ausgebildet ist.

5. Instrument nach Anspruch 1 oder 2, dadurch gekennzeichnet, daß die Schneidelektrode (14) am proximalen Ende um eine Querachse (22) schwenkbar angeordnet und über ein Getriebe (78), vorzugsweise aus einem Stift (76) und einer Kulissenbahn (75) bestehend, von dem axial bewegbaren Leitungsstab (17) zu einer Schneid-Schwenkbewegung antreibbar ist.

6. Instrument nach Anspruch 5, dadurch gekennzeichnet, daß die Schneidelektrode (14) scherenartig von einer Position außerhalb der Koagulationselektroden (12, 13) in eine Position zwischen den Koagulationselektroden (12,13) verschwenkbar ist.

7. Instrument nach Anspruch 5 oder 6, dadurch gekennzeichnet, daß die Schneidelektrode (14) einen Drahtbügel (14') mit einem zwischen die Koagulationselektroden (12, 13) bringbaren, vorzugsweise zumindest annähernd geradlinigen oder wellenförmigen sowie im eingeschwenkten Zustand zwischen den Koagulationselektroden (12, 13) parallel zu diesen verlaufenden Schneidenteil (14'') ausgebildet ist.

8. Instrument nach Anspruch 7, dadurch gekennzeichnet, daß der Drahtbügel (14') an einem vorzugsweise parallel zum Schneidenteil (14'') verlaufenden Tragarm (14''') angeordnet ist, der schwenkbar gelagert ist.

9. Instrument nach einem der Ansprüche 5 bis 8, dadurch gekennzeichnet, daß ein isolierendes Lager (23) sich zwischen den Koagulationselektroden (12, 13) in deren hinterem Bereich erstreckt.

10. Instrument nach einem der vorhergehenden Ansprüche, dadurch gekennzeichnet, daß das innere Leitungsrohr (16) hinten weiter vorsteht als das äußere Leitungsrohr (15) und daß der feststehende Kopfteil (21) des Betätigungskopfes (19) eine Aufnahmebohrung (24) für das distale Ende des Instrumentenschafts (11) und dahinter jeweils mit einem der Leitungsrohre (15, 16) ausgerichtete und zu diesen komplementäre Kontaktkanäle (25,26) aufweist, die mit dem Kabelanschluß (18) elektrisch verbunden sind.

11. Instrument nach Anspruch 10, dadurch gekennzeichnet, daß der Leitungsstab (17) hinten über das innere Leitungsrohr (16) vorsteht und daß axial ausgerichtet mit den Kontaktkanälen (25, 26) im beweglichen Kopfteil (21) eine Befestigungsvorrichtung (27) für das hintere Ende des Leitungsstabes (17) vorgesehen ist.

12. Instrument nach einem der vorhergehenden Ansprüche, dadurch gekennzeichnet, daß die Kopfteile (20, 21) axial miteinander ausgerichtet im wesentlichen hintereinander und bevorzugt teleskopisch ineinander verschiebbar angeordnet sind.

13. Instrument nach einem der vorhergehenden Ansprüche, dadurch gekennzeichnet, daß die Kopfteile (20, 21) voneinander trennbar sind.

14. Instrument nach Anspruch 11 und 13, dadurch gekennzeichnet, daß sich zwischen dem beweglichen Kopfteil (21) und dem feststehenden Kopfteil (20) exzentrisch angeordnete Kontaktstifte (28, 29) axial erstrecken, die in einem Kopfteil (21) fest und im anderen (20) in Kontaktbohrungen (30, 31) axial gleitbar angeordnet sind, und daß die Befestigungsvorrichtung (27) für den Leitungsstab (17) lösbar ist.

15. Instrument nach Anspruch 14, dadurch gekennzeichnet, daß die Kontaktstifte (28, 29) exzentrisch in einem Isolierblock (32) fest angeordnet sind, der gleichzeitig einen Teil der Befestigungsvorrichtung (27) für den Leitungsstab (17) bildet.

16. Instrument nach einem der vorhergehenden Ansprüche, dadurch gekennzeichnet, daß am distalen Ende des beweglichen Kopfteils (21) eine elektrische Steckverbindung (33) mit wenigstens drei Kontakten vorgesehen ist.

## Claims

1. Medical high frequency coagulation-cutting instrument comprising an instrument shaft (11) at the proximal end of which there are provided two stationary coagulation electrodes (12, 13) and a movable cutting electrode (14); and also an actuating head (19) arranged at the distal end of the instrument (11) and consisting of a fixed head part (20) and a head part (21) which is movable relative thereto, wherein a relative movement of the two head parts (20, 21) is transmitted to the cutting electrode (14), wherein the coagulation electrodes (12, 13) and also the cutting electrode (14) lead via high frequency feedlines (15, 16, 17), which are laid insulated relative to one another in the instrument shaft (11), to a cable connection (18) which is preferably provided at the distal end for the purpose of connection to a high frequency supply device, wherein the high frequency supply device has at least two switch positions, in one of which the cutting electrode (14) is switched off and the coagulation electrodes (12, 13) are energised with a high frequency coagulation current and in the other of which one or both of the coagulation electrodes (12, 13) are connected as neutral electrodes and a high frequency voltage which enables a cutting arc is applied to the cutting electrode (14) and the neutral electrode, wherein the high frequency supply line for the one coagulation electrode is a conductive tube within which the high frequency supply line for the cutting electrode (14) is arranged in an insulated manner as an axial conductive bar (17) axially movable through the actuating head (19), characterised in that the high frequency supply line for the second coagulation electrode is likewise formed as a second conductive tube surrounding the conductive bar (17) and both conductive tubes (15, 16) are arranged inside one another and are preferably arranged concentric to one another and to the instrument shaft (11).

2. Instrument in accordance with claim 1, characterised in that the coagulation electrodes are formed as two hook electrodes (12, 13) which extend at least substantially parallel to one another, with the cutting electrode (14) being movably arranged between the hook electrodes.

3. Instrument in accordance with claim 1 or claim 2, characterised in that the cutting electrode (14) is secured to the proximal end of the conductive bar (17) and is axially displaceable relative to the coagulation electrodes (12, 13) through the conductive bar (17) which is displaceable by means of the actuating head (19).

4. Instrument in accordance with claim 1, characterised in that the cutting electrode (14) is formed as a wire loop, in particular of rhombic wire.

5. Instrument in accordance with claim 1 or claim 2, characterised in that the cutting electrode (14) is pivotally arranged at the proximal end about a transverse axis (22) and is drivable by the axially movable conductive bar (17) to execute a pivotal cutting movement via a transmission (78) which preferably comprises a pin (76) and a cam-track (75).

6. Instrument in accordance with claim 5, characterised in that the cutting electrode (14) is pivotable in scissors-like manner from a position outside of the coagulation electrodes (12, 13) into a position between the coagulation electrodes (12, 13).

7. Instrument in accordance with claim 5 or claim 6, characterised in that the cutting electrode (14) is formed as a wire hoop (14'), with a cutting part (14'') which can be brought between the coagulation electrodes (12, 13), which is preferably at least approximately of straight-line or of wave shape and which also extends in the inwardly pivoted state between the coagulation electrodes (12, 13) parallel to the latter.

8. Instrument in accordance with claim 7, characterised in that the wire hoop (14') is arranged on a carrying arm (14''') which preferably extends parallel to the cutting part (14'').

9. Instrument in accordance with one of the claims 5 to 8, characterised in that an insulating bearing (23) extends between the coagulation electrodes (12, 13) in their rear region.

10. Instrument in accordance with claim 1, characterised in that the inner conductive tube (16) projects further rearwardly than the outer conductive tube (15); and in that the fixed head part (21) of the actuating knob (19) has a receiving bore (24) for the distal end of the instrument shaft (11) and behind this, has respective contact channels (25, 26) which are each aligned with one of the conductive tubes and complementary to the latter, by means of which they are electrically connected to the cable connection (18).

11. Instrument in accordance with claim 10, characterised in that the conductive bar (17) projects rearwardly beyond the inner conductive tube (16); and in that a mounting device (27) for the rear end of the conductive bar (17) is provided in the movable head part (21) axially aligned with the contact channels (25, 26).

12. Instrument in accordance with any one of the preceding claims, characterised in that the head parts (20, 21) are axially aligned with one another, essentially behind one another, and are preferably telescopically displaceable within one another.

13. Instrument in accordance with any one of the preceding claims, characterised in that the head parts (20, 21) are separable from one another.

14. Instrument in accordance with claim 11 and claim 13, characterised in that eccentrically arranged contact pins (28, 29) extend axially between the movable head part (21) and the stationary head part (20), are fixedly arranged in the one head part (21) and are axially slideably arranged in the other head part (20) in contact bores (30, 31); and in that the securing device (27) for the conductive bar (17) is releasable.

15. Instrument in accordance with claim 14, characterised in that the contact pins (28, 29) are eccentrically and fixedly arranged in an insulating block (32) which simultaneously forms a part of the securing device (27) for the conductive bar (17).

16. Instrument in accordance with claim 1, characterised in that an electrical plug connection (32) with at least three contacts is provided at the distal end of the movable head part (21).

## Revendications

1. Appareil chirurgical pour la coagulation et la coupe à haute fréquence doté d'une tige (11), sur l'extrémité proximale de laquelle sont prévues deux électrodes de coagulation (12, 13) fixes et une électrode de coupe (14) mobile, et d'un organe supérieur de commande (19) disposé sur l'extrémité distale de la tige (11), lequel organe supérieur comprend une partie supérieure (20) fixée sur la tige (11) et une partie supérieure (21) mobile par rapport à la première, moyennant quoi un mouvement relatif des deux parties supérieures (20, 21) est transmis à l'électrode de coupe (14), les électrodes de coagulation (12, 13) ainsi que l'électrode de coupe (14) sont guidées vers un branchement de câble (18) prévu de préférence sur l'extrémité distale pour une liaison avec un appareil d'alimentation de haute fréquence au moyen des lignes d'arrivée de haute fréquence (15, 16, 17) posées dans la tige de l'appareil (11) et isolées entre elles et l'appareil d'alimentation en haute fréquence présente au moins deux positions de commutation, dans l'une desquelles l'électrode de coupe (14) est déconnectée et les électrodes de coagulation (12, 13) sont alimentées avec un courant de haute fréquence pour coagulation, et dans l'autre desquelles une ou deux électrodes de coagulation (12, 13) sont branchées comme électrode neutre, et une tension de haute fréquence permettant un arc de coupe est appliquée sur l'électrode de coupe (14) et l'électrode neutre, la ligne d'arrivée de haute fréquence pour la une électrode de coagulation étant un tuyau de conduite, à l'intérieur duquel la ligne d'arrivée de haute fréquence pour l'électrode de coupe (14) est isolée et se présente sous la forme d'une barre de conduite axiale et mobile axialement au moyen de l'organe supérieur de commande (19), caractérisé en ce que la ligne d'arrivée de haute fréquence pour la deuxième électrode de coagulation est également conçue comme un deuxième tuyau de conduite entourant la barre de conduite (17) et deux tuyaux de conduite (15, 16) sont emboîtés l'un dans l'autre et disposés de préférence de façon concentrique l'un par rapport à l'autre et par rapport à la tige de l'appareil (11).

2. Appareil selon la revendication 1, caractérisé en ce que les électrodes de coagulation sont réalisées comme deux électrodes en crochet (12, 13) au moins sensiblement parallèles entre elles, entre lesquelles l'électrode de coupe (14) est disposée de façon mobile.

3. Appareil selon la revendication 1 ou 2, caractérisé en ce que l'électrode de coupe (14) est fixée sur l'extrémité proximale de la barre de conduite (17) et peut être déplacée axialement par rapport aux électrodes de coagulation (12, 13) au moyen de la barre de conduite (17) déplaçable au moyen de l'organe supérieur de commande (19).

4. Appareil selon l'une quelconque des revendications précédentes, caractérisé en ce que l'électrode de coupe (14) est conçue comme un portefil, et en particulier comme un fil en forme de losange.

5. Appareil selon la revendication 1 ou 2, caractérisé en ce que l'électrode de coupe (14) est disposée sur l'extrémité proximale de façon à pouvoir pivoter autour d'un axe transversal (22) et peut être entraînée au moyen d'un engrenage (78), comprenant de préférence une broche (76) et une glissière coulissante (75), par la barre de conduite (17) mobile axialement en un mouvement pivotant de coupe.

6. Appareil selon la revendication 5, caractérisé en ce que l'électrode de coupe (14) peut pivoter à la façon de ciseaux d'une position à l'extérieur des électrodes de coagulation (12, 13) à une position entre les électrodes de coagulation (12, 13).

7. Appareil selon la revendication 5 ou 6, caractérisé en ce que l'électrode de coupe (14) est conçue comme un porte-fil (14') avec une partie de coupe (14'') pouvant être amenée entre les électrodes de coagulation (12, 13), de préférence au moins approximativement rectiligne ou ondulée et parallèle aux électrodes de coagulation (12, 13) lorsqu'elle est insérée entre celles-ci.

8. Appareil selon la revendication 7, caractérisé en ce que le porte-fil (14') est disposé sur un bras support (14''') de préférence parallèle à la partie de coupe (14''), lequel bras est logé de façon pivotante.

9. Appareil selon l'une quelconque des revendications 5 à 8, caractérisé en ce qu'un support (23) isolant s'étend entre les électrodes de coagulation (12, 13) dans leur zone arrière.

10. Appareil selon l'une quelconque des revendications précédentes, caractérisé en ce que le tuyau de conduite (16) intérieur dépasse à l'arrière d'avantage que le tuyau de conduite (15) extérieur et que la partie supérieure (21) fixe de l'organe supérieur de commande (19) présente un perçage de positionnement (24) pour l'extrémité distale de la tige d'appareil (11) et derrière des canaux de contact (25, 26) alignés respectivement avec l'un des tuyaux de conduite (15, 16) et complémentaires à ceux-ci, lesquels canaux sont reliés électriquement au branchement de câble (18).

11. Appareil selon la revendication 10, caractérisé en ce que la barre de conduite (17) dépasse à l'arrière du tuyau de conduite intérieur et un dispositif de fixation (27) pour l'extrémité arrière de la barre de conduite (17) est prévu dans la partie supérieure (21) mobile, aligné axialement avec les canaux de contact (25, 26).

12. Appareil selon l'une quelconque des revendications précédentes, caractérisé en ce que les parties supérieures (20, 21) sont alignées entre elles sur un plan axial, sont disposées sensiblement les unes derrières les autres et sont emboîtées de préférence les unes dans les autres.

13. Appareil selon l'une quelconque des revendications précédentes, caractérisé en ce que les parties supérieures (20, 21) peuvent être détachées les unes des autres.

14. Appareil selon les revendications 11 et 13, caractérisé en ce qu'entre la partie supérieure (21) mobile et la partie supérieure (20) fixe se trouve sur un plan axial des broches de contact (28, 29) excentrées qui sont disposées de façon fixe dans une partie supérieure (21) et de façon coulissante axialement dans l'autre (20) dans des perçages de contact (30, 31), et en ce que le dispositif de fixation (27) pour la barre de conduite (17) est amovible.

15. Appareil selon la revendication 14, caractérisé en ce que les broches de contact (28, 29) sont fixes et excentrées dans un bloc isolant (32) qui forme en même temps une partie du dispositif de fixation (27) pour la barre de conduite (17).

16. Appareil selon l'une quelconque des revendications précédentes, caractérisé en ce qu'un connecteur (33) électrique avec au moins trois contacts est prévu sur l'extrémité distale de la partie supérieure (21) mobile.
